# EUROPÄISCHE PATENTSCHRIFT

(11) **EP 3 151 723 B1**
(45) Veröffentlichungstag und Bekanntmachung des Hinweises auf die Patenterteilung: **07.11.2018**
(21) Anmeldenummer: 16728699.6
(22) Anmeldetag: 13.06.2016
(51) Int. Cl.: A61B 1/00, A61B 1/015, A61B 1/018

(54) **ENDOSKOP MIT MEHREREN PROXIMALEN ARBEITSKANAL-ANSCHLÜSSEN**
ENDOSCOPE HAVING MULTIPLE WORKING CHANNEL CONNECTIONS
ENDOSCOPE POURVU D'UN PLURALITÉ DE RACCORDS DE CONDUITS DE TRAVAIL PROXIMAUX

(30) Priorität: 14.08.2015 DE 102015113428
(43) Veröffentlichungstag der Anmeldung: 12.04.2017
(73) Patentinhaber: invendo medical GmbH, 86438 Kissing (DE)
(72) Erfinder: FIEBER, Markus, 80636 München (DE); ABICHT, Felix, 82337 Walchstadt (DE); SCHÜTZ, Günter, 86152 Augsburg (DE)
(74) Vertreter: Winter, Brandl, Fürniss, Hübner, Röss, Kaiser, Polte - Partnerschaft mbB
(86) Internationale Anmeldenummer: PCT/EP2016/063519
(87) Internationale Veröffentlichungsnummer: WO 2017/028981

(56) Entgegenhaltungen:
- EP-A1- 2 604 171
- US-A1- 2005 256 376

## Beschreibung

Die vorliegende Erfindung betrifft ein Endoskop (einschließlich Koloskop und/oder Gastroskop) mit Optik, Beleuchtung sowie einem Arbeitskanal mit Endoskopspezifischer Funktion und Dimensionierung, der in einem vorzugsweise flexiblen Endoskopschaft ausgebildet ist und sich an der distalen Endoskopspitze zur Umgebung hin öffnet.

### Hintergrund der Erfindung

Endoskope sind medizinische Arbeitsgerätschaften, mittels denen das Innere von in der Regel lebenden Organismen, aber auch technischen Hohlräumen untersucht und/oder manipuliert werden kann. Ursprünglich für die humanmedizinische Diagnostik entwickelt, werden sie heute auch für minimal-invasive operative Eingriffe an Mensch und Tier sowie in der Industrie zur Sichtprüfung schwer zugänglicher Hohlräume eingesetzt. Zu den Endoskopen zählen die starren und flexiblen Endoskope und deren Unterarten.

Ein starres Endoskop leitet die Bildinformationen des zu untersuchenden Objekts bzw. Raums durch ein Linsensystem im Inneren des Endoskopschafts bzw. im distalen Endoskopkopf an ein proximal angeordnetes Okular weiter. Beispiele hierfür sind das technische Boreskop und med. das Arthroskop und Zystoskop.

Das für die Untersuchung/Inspektion notwendige Licht wird entweder durch ein distales Leuchtmittel (z.B. LED) im Endoskopkopf bereitgestellt oder über einen an ein proximales Leuchtmittel angeschlossenen Lichtleiter, ebenfalls im Inneren des Schafts durch Glasfaserbündel an die Spitze des Endoskops transportiert.

Bei einem flexiblen Endoskop werden Bild und Licht ebenfalls häufig über Glasfaserbündel übertragen. Beispiele sind das technische Flexoskop und das med. Gastroskop, Koloskop und Bronchoskop.

Ab einem praktikablen Durchmesser sind flexible (und auch starre) Endoskope auch mit auswechselbaren statt festmontierten Objektiven (*Vor*/*Seit* oder *Rückwärts*) sowie zusätzlichen Arbeitskanälen zum Einführen von mikromechanischen Geräten (kleine Zangen oder Greifer) in den Untersuchungs- bzw. Inspektionsraum erhältlich. Flexible Endoskope besitzen meist eine über eingebaute Bowdenzüge oder Hydraulik-Stellantriebe fernsteuerbare Gerätespitze (Endoskopkopf). Diese kann je nach Modell und Durchmesser nach 2 (*auf-ab*) oder nach 4 Seiten (*auf-ab* und *rechts-links*) teilweise bis zu 180° abgewinkelt werden. Die Länge dieser abwinkelbaren Spitze kann je nach Durchmesser zwischen 30 und 70 mm liegen. Im proximalen Handgriff des Geräts/Endoskops ist eine Mechanik eingebaut, die über Kippbügel oder Handräder auf die Bowdenzüge/Stellantriebe einwirkt und die Bewegung der Spitze ermöglicht.

Die jüngste Unterart der flexiblen Endoskope bilden die Videoendoskope, oft auch Videoskope genannt. Videoendoskope eröffnen ein neues Kapitel in der modernen Endoskopie, da sie zur Bilderzeugung und -übertragung digitale Technologien nutzen. Ein am distalen Objektiv des Videoendoskops angebrachter CCD- bzw. CMOS-Chip erzeugt ein digitales Bild des Untersuchungsobjekts und leitet es an die folgenden Baugruppen des Videoendoskops weiter. Meist bereitet dann ein Prozessor diese Daten auf, sendet sie zur Ausgabe an einen Monitor, oder legt sie auf einer Festplatte oder anderen Datenspeichern ab. Videoendoskope ermöglichen je nach Ausstattungsvariante das Einfrieren und Speichern von Bildern sowie mehrstündige Videoaufzeichnungen für eine nachträgliche Aufbereitung/Optimierung oder auch das Vermessen eines Bildes bzw. Objektes. Auch diese Gerätetechnik besitzt eine fernsteuerbar, abwinkelbare Gerätespitze, nach 2 oder 4 Richtungen. Diese können mechanisch oder elektronisch/hydraulisch gesteuert werden. Die mechanische Steuerung erfolgt über ein kleines Getriebe über Kipphebel oder Drehräder. Einige Videoskope haben anstelle der Mechanik kleine Elektromotoren oder Hydraulikbalge eingebaut, die beispielsweise über einen Joystick ansteuerbar sind.

Neuerdings kommen bei Videoskopen anstelle der externen Lichtprojektoren, auch LED-Leuchtkörper zum Einsatz. Diese können in der Gerätespitze eingebaut sein, oder auch im proximalen Handgriff. Im letzteren Fall wird dann das Licht über eingebaute Glasfasern nach vorn zur Spitze geleitet.

Zusammenfassend sind demnach starre wie auch flexible Endoskope der einschlägigen Gattung (einschließlich des vorliegenden Erfindungsgegenstands) gekennzeichnet durch einen Endoskopschaft (flexibel oder starr), an dessen distalem Ende (Endoskopkopf) eine Beleuchtung (Lichtaustritt eines Lichtleiterkabels oder ein Leuchtkörper) sowie eine Optik (Linsensystem und/oder lichtempfindlicher Chip) angeordnet sind. Innerhalb des Endoskopschafts ist (neben optionalen diversen Funktions-/Versorgungskanälen beispielsweise für die Optik, die Beleuchtung und/oder die Abkrümmeinrichtung der Instrumentenspitze) ein Arbeitskanal ausgeformt, der dafür vorgesehen und angepasst ist, chirurgische Instrumente durch das Endoskop hindurch in den Patientenkörper einzuführen, um unter visueller Kontrolle über die Optik und die Beleuchtung (minimalinvasive) Operationen/Behandlungen vorzunehmen.

Da chirurgische Instrumente für minimalinvasive Eingriffe eine Mindestgröße aufweisen, hat der Arbeitskanal einen entsprechenden Durchmesser, der das Einführen der chirurgischen Instrumente erlaubt. Dieser Durchmesser ist in der Regel deutlich größer als der der Funktions-/Versorgungskanäle.

Neben der vorstehend beschriebenen Funktion hat der Arbeitskanal aber auch die Aufgabe, beispielsweise Behandlungsflüssigkeiten (Medikamentlösungen, etc.) zu fördern oder Patientengewebe und/oder Körperflüssigkeiten abzutransportieren. Außerdem kann über den Arbeitskanal Spülflüssigkeit an die Behandlungsstelle im Patientenkörper gepumpt werden.

### Stand der Technik

Aus dem Stand der Technik sind allgemein Endoskope der vorstehenden Gattung (starre und flexible Endoskope) bekannt, bei denen der Arbeitskanal sowohl an der distalen Endoskopspitze wie auch im Bereich des proximalen Handgriffs jeweils eine Austrittsöffnung hat. Über die proximale Öffnung kann dann beispielsweise ein chirurgisches Instrument eingeführt werden oder es kann eine Druck-/Saugpumpe angeschlossen werden.

Die US 2005/0256376 A1 offenbart einen Konnektor für ein Endoskop, mit dem eine Fluidverbindung zwischen Kanälen eines mehrlumingen Endoskop-Schlauchs und mehreren Versorgungsschläuchen für Wasser, Luft aber auch einer Absaugung, hergestellt werden kann.

Das Problem dieser Endoskope besteht jedoch darin, dass insbesondere bei minimalinvasiven chirurgischen Eingriffen bei in den Arbeitskanal eingeführtem chirurgischem Instrument gleichzeitig beispielsweise Spüllösung an die Operationsstelle herangeführt werden muss. Dies erfolgt dann über einen zum Arbeitskanal separat im Endoskopschaft ausgebildeten Versorgungskanal mit vergleichsweise kleinem Durchmesser. Es hat sich hier aber gezeigt, dass nur wenig Spüllösung über diesen engen Versorgungskanal pro Zeiteinheit gefördert werden kann, was sich insbesondere bei besonders langen Endoskopschäften (bis zu 250cm Länge) insoweit negativ auswirkt, als dass die Spüllösung mit hohem Druck in den Versorgungskanal gepumpt werden muss, um dieses in ausreichender Menge pro Zeiteinheit bis zur distalen Endoskopspitze fördern zu können.

### Kurzbeschreibung der Erfindung

Angesichts dieser, von der vorliegenden Anmelderin erkannten Problematik ist es die Aufgabe der Erfindung, ein Endoskop dieser Gattung (vorzugsweise flexibel aber ggf. auch starr) bereitzustellen, mittels dem eine ausreichende Flüssigkeitsver- und/oderentsorgung auch bei gleichzeitig in den Arbeitskanal eingeführtem chirurgischen Instrument möglich ist.

Ferner soll vorzugsweise die Handhabung des Endoskops verbessert werden, indem auf einfache Weise Teile des Endoskops als Ein-Weg-Artikel nach Gebrauch einfach entsorgt und andere Teile des Endoskops als Mehr-Weg-Artikel nach Gebrauch auf einfache Weise gereinigt werden können. Schließlich soll weiter vorzugsweise der Betrieb des Endoskops verbessert werden.

Diese Aufgabe wird erfindungsgemäß durch ein Endoskop mit den Merkmalen des Anspruchs 1 gelöst. Vorteilhafte Ausgestaltungen der Erfindung sind Gegenstand der Unteransprüche.

Mit anderen Worten betrifft die Erfindung also ein Endoskop mit einem Endoskopschaft, an dessen distalem Ende zumindest eine Optik sowie eine Beleuchtung vorgesehen sind, die über Funktions- und/oder Versorgungskanäle innerhalb des Endoskopschafts an eine Betriebseinheit oder Betriebsstation angeschlossen oder anschließbar sind und einem am distalen Ende des Endoskopschafts sich öffnenden Arbeitskanal, der im Endoskopschaft ausgeformt und dafür angepasst ist, chirurgischen Instrumente der Minimalinvasivbauart längsverschieblich aufzunehmen, wobei der Arbeitskanal am proximalen Endbereich des Endoskops und vorzugsweise des Endoskopschafts zumindest zwei separate, unabhängig voneinander nutzbare Anschlüsse hat, von denen zumindest ein erster Anschluss dazu geeignet und vorzugsweise dazu angepasst ist, um darüber das chirurgische Instrument in den Arbeitskanal einzuführen und von denen zumindest ein zweiter Anschluss dazu geeignet und vorzugsweise dazu angepasst ist, um ein Fluid in den Arbeitskanal proximal einzuleiten oder aus diesem zu entnehmen. Erfindungsgemäß hat der Endoskopschaft einen proximalen Anschlusseinsatz, an dem die proximalen Arbeitskanal-Anschlüsse ausgebildet sind, wobei der Anschlusseinsatz einen Rohrlauf hat, dessen eines Ende mit dem Arbeitskanal innerhalb des mit dem Anschlusseinsatz fest verbundenen biegeflexiblen Schaftabschnitts fluidverbunden ist und an dessen anderem Ende ein Anschlussstutzen angeordnet ist mit einer Anzahl von Anschlussrohren, welche sämtlich in den Rohrlauf einmünden und welche die proximalen Arbeitskanal-Anschlüsse bilden. Die Funktions- und/oder Versorgungskanäle sind dabei unter Umgehung eines Handgriffs des Endoskops an die Betriebseiheit oder Betriebsstation angeschlossen oder anschließbar.

Ein Kerngedanke der Erfindung ist es demnach, den Arbeitskanal zum Zwecke des Einführens von chirurgischen Instrumenten aber auch des Zu- und/oder Abführens von Fluiden/Flüssigkeiten besser zu nutzen. Die gegenüber dem Stand der Technik verbesserte Ausnutzung des Arbeitskanals wird erfindungsgemäß dadurch erreicht, indem dem Arbeitskanal mindestens zwei proximale Anschlüsse zugeordnet sind, welche separat und unabhängig voneinander (damit auch parallel/gleichzeitig) genutzt werden können, um jeweils ein Medium (ein chirurgisches Instrument und/oder ein Fluid) über jeden proximalen Anschluss in den Arbeitskanal (parallel/gleichzeitig) ein- und/oder abzuführen.

In anderen Worten ausgedrückt hat der (einzige) Arbeitskanal des Endoskops wenigstens zwei proximale Anschlüsse, von denen wenigstens ein proximaler Anschluss für das Einführen eines (medizinischen) Gegenstands wie chirurgisches Instrument geeignet und vorzugsweise angepasst ist und wenigstens ein anderer proximaler Anschluss für das Einführen/Entnehmen eines Fluids wie eine Flüssigkeit (z.B. Spüllösung, Körperflüssigkeit, etc.) und/oder ein Gas (z.B. Luft zum Entfalten eines Patientendarms) geeignet und vorzugsweise angepasst ist.

Die mehreren Anschlüsse des Arbeitskanals werden durch einen vorzugsweise einstückigen Anschlusseinsatz gebildet. Dieser Anschlusseinsatz hat (besteht) einen Rohrlauf, dessen eines Ende mit dem Arbeitskanal (fluid-)verbunden (verbindbar) ist und an dessen anderem Ende ein Anschlussstutzen angeordnet ist mit einer Anzahl von Anschlussrohren, die sämtlich in den Rohrlauf einmünden. Vorzugsweise ist der Anschlusseinsatz fest mit dem Endoskopschaft an dessen proximalem Ende verbunden. Sofern der Endoskopschaft biegeflexibel ist, weist der Anschlusseinsatz deutlich starrere (steifere) Eigenschaften auf.

Auf diese Weise kann der Arbeitskanal auch bei eingeschobenem/eingeführtem chirurgischen Instrument gleichzeitig zur Zufuhr und/oder Entnahme von Fluid genutzt werden. Da folglich ein im Stand der Technik hierfür gesondert vorgesehener Ver- / Entsorgungskanal entfallen kann, lässt sich der Arbeitskanal vergleichsweise großzügig dimensionieren, ohne dass der Endoskoschaft-Außendurchmesser hierfür aufgeweitet werden müsste.

Die Anpassung des einen Anschlusses für das Einführen eines chirurgischen Instruments definiert sich im einfachsten Fall im Anschluss-Inndurchmesser, der so groß sein muss, dass ein chirurgisches Instrument hineingeschoben werden kann. In anderen Worten ausgedrückt orientiert sich der Anschluss-Durchmesser am Durchmesser des Arbeitskanals. Außerdem sollte dieser Anschluss vorzugsweise einen sanften Eingang zum Arbeitskanal aufweisen, also etwa einen inneren Bogen ausführen oder axial zum Arbeitskanal verlaufen, damit das einzuführende chirurgische Instrument nicht abgeknickt wird.

Die Anpassung des Anschlusses für das Ein-/Ausführen eines Fluids definiert sich hingegen im einfachsten Fall durch die Ausbildung einer Verschlussmöglichkeit. In anderen Worten ausgedrückt hat dieser Anschluss eine zusätzliche Einrichtung, an welcher beispielsweise ein Schlauch (fluiddicht) angeschlossen werden kann. Z.B. kann diese ein Rohrstutzen/Nippel sein, auf welchem ein Schlauch aufgesteckt werden kann, oder ein Bajonett-, Schraub- oder Lueranschluss.

Vorteilhafter Weise ist der Rohrlauf dafür angepasst, in einen Handgriff eingesetzt zu werden, derart, dass die Anschlussrohre des Anschlussstutzens zumindest abschnittsweise aus dem Handgriff herausragen, bzw. von außen zugänglich sind. Der Handgriff ist somit von den Anschlüssen separiert und wird daher nicht oder nur geringfügig kontaminiert.

Gemäß einem ggf. unabhängig zu beanspruchenden Aspekt der vorliegenden Erfindung ist der Handgriff als (lösbarer) Aufsteckhandgriff ausgebildet, der vorzugsweise mit dem Anschlusseinsatz und weiter vorzugsweise mit dem Rohrlauf des Anschlusseinsatzes weiter vorzugsweise klemmend zusammenwirkt. D.h. das erfindungsgemäße Endoskop besteht im Wesentlichen aus zwei Baueinheiten, nämlich dem Endoskopschaft (mit dem vorzugsweise flexiblen, in den Patientenkörper einzuführenden Schaftabschnitt mit Endoskopkopf und den darin verbauten Funktionen sowie mit dem vorzugsweise starren Anschlusseinsatz) und dem separat hierzu ausgebildeten Handgriff, der einfach am Endoskopschaft (Anschlusseinsatz) temporär fixiert werden kann.

Hierfür kann der Rohrlauf des Anschlusseinsatzes eine Anzahl von Rastelementen oder Hinterschneidungen haben, die mit entsprechenden Rastelementen oder Hinterschneidungen auf Seiten des Handgriffs zusammenwirken. Vorzugsweise bildet der Handgriff einen Clip-/Schalenabschnitt mit einer Anzahl von Hinterschneidungen und/oder Vorsprüngen, derart, dass der Handgriff auf den Rohrlauf aufgeclipt werden kann, wobei entsprechende Vorsprünge und/oder Hinterschneidungen auf Seiten des Clipabschnitts mit den Hinterschneidungen und/oder Vorsprüngen am Rohrlauf in Formschluss kommen. Dadurch wird ein sicherer (aber lösbarer) Halt des Handgriffs auf dem Rohrlauf gewährleistet.

Wie vorstehend bereits angedeutet wurde, ist der Anschlusseinsatz vorzugsweise fest mit dem für das Einführen in den Patientenkörper vorgesehenen (biegeflexiblen) Endoskopschaftabschnitt gekoppelt. Der Endoskopschaft (einschließlich des Anschlusseinsatzes) ist hierbei vorzugsweise als Einweg-Gegenstand konzipiert. Da aber der Handgriff keinen unmittelbaren Kontakt beispielsweise mit Körperflüssigkeit des Patienten bekommt (sämtlich Leitungen und Anschlüsse sind im Endoskopschaft), kann dieser als Mehrweg-Produkt vorgesehen sein. Daher ist die Kopplung zwischen Handgriff und Anschlusseinsatz lösbar, vorliegend vorzugsweise als Clickverbindung gedacht. Es könnte aber jedwede andere lösbare Kopplung wie Einsteck-, Klett, Spann- oder Schraubverbindung vorgesehen sein.

Vorzugsweise sind am Anschlusseinsatz weitere Anschlüsse für die Versorgungs-/Funktionskanäle und/oder die Funktionen wie Optik, Beleuchtung, Stellantriebe, etc. ausgebildet. Auf diese Weise, werden alle Funktionen des Endoskopschafts einschließlich der im Endoskopkopf verbauten Funktion wie Optik, Beleuchtung, Stellantriebe, etc. unmittelbar am Anschlusseinsatz unter Umgehung des Handgriffs angeschlossen.

Gemäß einem anderen, ggf. unabhängig beanspruchbaren Aspekt der vorliegenden Erfindung hat der Handgriff eine Betätigungseinrichtung, beispielsweise ein Tastenfeld zur Aktuierung einer (separaten) Betriebsstation, welche an das Endoskop über die vorstehend genannten weiteren Anschlüsse angeschlossen/anschließbar ist. Die Steuerungs-Verbindung zwischen Handgriff und Betriebsstation erfolgt vorzugsweise kabellos insbesondere per W-Lan, Blue-Tooth oder Infrarot-Signal, wobei natürlich aber auch eine Kabelverbindung vorgesehen sein kann.

Weiter vorzugsweise ist am Handgriff ein Sensor oder dergleichen Erfassungsschalter angebracht, der den Anschlusseinsatz erfasst, wenn dieser mit dem Handgriff gekoppelt ist, um daraufhin die Aktuierung der Betriebsstation freizuschalten. Zusätzlich oder alternativ kann an den weiteren Anschlüssen ein ähnlicher Sensor/Erfassungsschalter mit gleicher Funktion und Wirkung angeordnet sein.

Gemäß einem anderen, ggf. unabhängig beanspruchbaren Aspekt der vorliegenden Erfindung bildet das Gehäuse des Handgriffs eine fluiddichte Ummantelung sowohl des Tastenfelds wie auch der darin aufgenommenen Elektronik. Im Fall einer Kabelverbindung ist auch der Anschluss zwischen Kabel und Handgriff vorzugsweise fluiddicht ummantelt. Der Handgriff bildet somit eine fluiddichte Einheit, die separat zum Endoskopschaft auf einfache Weise, beispielsweise durch ein Tauchverfahren, gereinigt werden kann.

### Figurenbeschreibung

Die vorliegende Erfindung wird nachfolgend anhand eines bevorzugten Ausführungsbeispiels unter Bezugnahme auf die begleitenden Figuren näher erläutert.
Fig. 1 zeigt einen Handgriff sowie einen Anschlusseinsatz eines Endoskopschafts gemäß einem bevorzugten Ausführungsbeispiel der Erfindung und
Fig. 2 zeigt den Handgriff und den Anschlusseinsatz gemäß Fig. 1 in gekoppeltem Zustand.

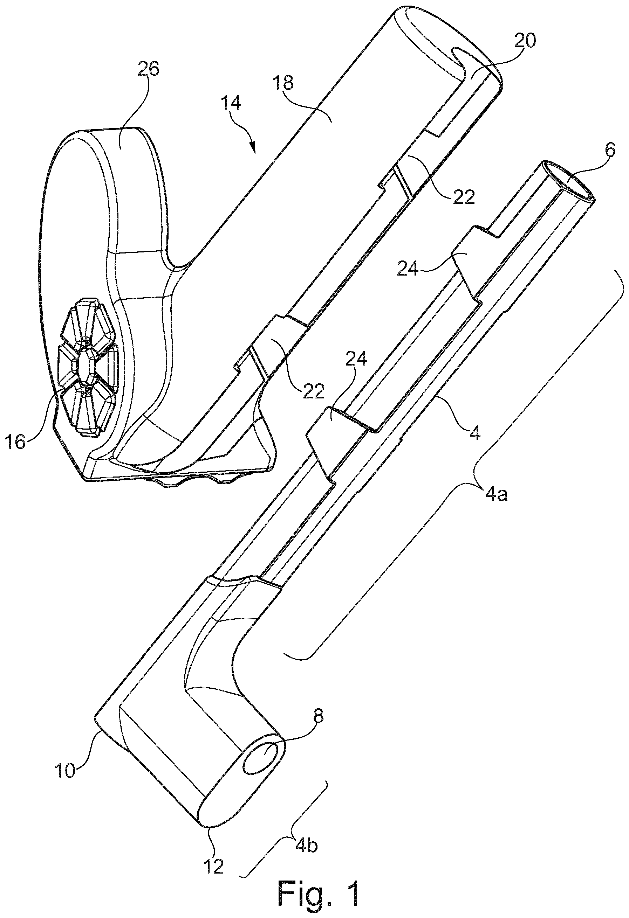

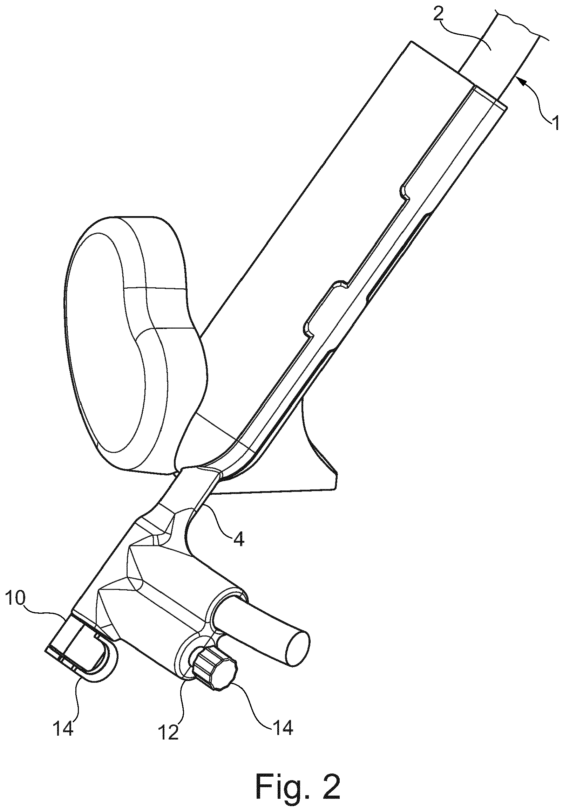

Gemäß der beiden Fig. 1 und 2 hat das erfindungsgemäße Endoskop einen Endoskopschaft 1, an dessen nicht weiter dargestellten distalen Endbereich (Endoskopkopf) eine Anzahl von Funktionen verbaut ist. Diese Funktionen betreffen u. a. eine Optik, eine Beleuchtung, ggf. einen aktiv per Stellantrieb betätigbaren Abwinklungsabschnitt unmittelbar vor dem Endoskopkopf, und dergleichen. Insoweit entspricht der Endoskopschaft der vorliegenden Erfindung dem bekannten Stand der Technik und braucht daher nicht näher beschrieben zu werden.

Im Unterschied zum allgemein bekannten Stand der Technik besteht der Endoskopschaft 1 der vorliegenden Erfindung aus einem biegeflexiblen Schaftabschnitt 2, der dafür geeignet und dazu angepasst ist, in den Hohlraum (z.B. Darm oder Speiseröhre, etc.) eines Patienten unter passiver Nachverfolgung von Hohlraumkrümmungen/-windungen eingeführt zu werden und einem proximal daran anschließenden Anschlusseinsatz 4, der fest (oder auch lösbar) mit dem biegeflexiblen Schaftabschnitt 2 (koaxial) in axialer Verlängerung zu diesem verbunden ist. Vorzugsweise besteht der Anschlusseinsatz 4 aus einem Material, das steifer ist als der biegeflexible Schaftabschnitt 2.

Innerhalb des Endoskopschafts 1 umfassend den flexiblen Schaftabschnitt 2 sowie den Anschlusseinsatz 4 ist ein (zentraler) Arbeitskanal 6 sowie eine Anzahl von Versorgungs-/Funktionskanälen (nicht weiter dargestellt) ausgebildet. In den Versorgungs-/Funktionskanälen sind zum Teil (elektrisch oder lichtleitende) Leitungskabel verlegt, die an die vorstehend genannten Funktionen angeschlossen sind. Es können aber auch Hydraulikflüssigkeit-führende Versorgungs-/Funktionskanäle vorgesehen sein, etwa zur hydraulischen Betätigung des Abwinklungsabschnitts oder zur Kühlung/Reinigung einzelner Funktionen im Endoskopkopf.

Diese Versorgungs-/Funktionskanäle erstrecken sich ausgehend vom Endoskopkopf durch den biegeflexiblen Schaftabschnitt 2 und den sich proximal daran anschließenden Anschlusseinsatz 4 und enden in einer Anschlusseinrichtung 8 bzw. Funktionsanschlüssen (z.B. kombinierte Elektrik/Hydraulik-Steckdose) am proximalen Ende des Anschlusseinsatzes 4.

Der Arbeitskanal 6 erstreckt sich ebenfalls ausgehend vom Endoskopkopf durch den biegeflexiblen Schaftabschnitt 2 und den Anschlusseinsatz 4 und hat einen Durchmesser, der das gleitende Hindurchführen von minimalinvasiven chirurgischen Instrumenten mit standardisierten Außendurchmessern (aus dem allgemeinen Stand der Technik bekannt) erlaubt. Der Arbeitskanal 6 öffnet sich am Endoskopkopf in Richtung distal zur Umgebung, sodass ein chirurgisches Instrument (Zange, Schere, Pinzette, etc.) aus dem Endoskopschaft vorausgeschoben werden kann.

Am proximalen Ende des Arbeitskanals mündet dieser in wenigstens zwei separate (Arbeitskanal-) Anschlüsse 10, 12, welche im Anschlusseinsatz 4 ausgebildet sind.

Im Konkreten hat der Anschlusseinsatz 4 neben den vorstehend beschriebenen Funktionsanschlüssen 8 wenigstens zwei weitere Arbeitskanalanschlüsse 10, 12, die separat sowie unabhängig voneinander (auch gleichzeitig) benutzt werden können.

Vorzugsweise ist zumindest einer der Anzahl von Arbeitskanalanschlüssen 10 dazu vorgesehen (angepasst), dass über diesen ein chirurgisches Instrument in den Arbeitskanal 6 eingeführt wird. Demzufolge hat dieser Arbeitskanalanschluss 10 wenigstens einen Durchmesser, der für das Einführen des chirurgischen Instruments (mit Standardaußendurchmesser) ausreicht. Vorzugsweise entspricht der Durchmesser dieses Arbeitskanalanschlusses dem des Arbeitskanals oder ist (etwas) größer. Des Weiteren ist dieser Arbeitskanalanschluss 10 bezüglich des Arbeitskanals 6 vorzugsweise so ausgerichtet, dass das chirurgische Instrument bei dessen Einführen in den Arbeitskanal nicht abknickt (in einem stumpfen Winkel oder axial zum Arbeitskanal 6).

Weiter vorzugsweise ist zumindest einer der Anzahl von Arbeitskanalanschlüssen 12 dazu vorgesehen (angepasst), dass über diesen ein Fluid in den Arbeitskanal 6 eingeführt oder abgeführt wird. Beispielsweise hat dieser Anschluss 12 eine Vorrichtung, die ein fluiddichtes Anschließen einer (nicht gezeigten) Pumpe, Spritze oder dergleichen ermöglicht. Eine solche Vorrichtung kann ein einfacher Rohrstutzen sein, auf den z.B. ein Schlauch aufgeschoben ist/wird oder ein Bajonett-, Schraub- oder Luerverschluss und dergleichen.

Jeder der Arbeitskanalanschlüsse 10, 12 kann ferner mit einem Verschluss (Deckel, Kappe, Blindstopfen, etc.) 14 versehen sein, mittels dem bei Nichtnutzung des jeweiligen Arbeitskanalanschlusses 10, 12 dieser fluiddicht verschlossen werden kann.

Vorzugsweise sind der eine Arbeitskanalanschluss 12 für das Ein-/Abführen von Fluid 12 und der Funktionsanschluss 8 in einem (stumpfen) Winkel (maximal 90°) zum Arbeitskanal 6 innerhalb des Anschlusseinsatzes 4 ausgerichtet. Weiter vorzugsweise verlaufen der eine Arbeitskanalanschluss 12 für das Ein-/Abführen von Fluid 12 und der Funktionsanschluss 8 parallel zueinander (auf einer Winkelposition bezüglich des Arbeitskanals 6). Sie können aber auch in Umfangsrichtung des Arbeitskanals versetzt zueinander angeordnet sein.

Insoweit bildet der Anschlusseinsatz 4 in seinem äußeren Erscheinungsbild quasi einen (distalen) Rohrlauf (in Verlängerung zum biegeflexiblen Schaftabschnitt) 4a und einen (proximalen) Anschlussstutzen 4b bestehend aus den Arbeitskanalanschlüssen 10, 12 und den weiteren (Funktions-) Anschlüssen 8 für die eingangs genannten Endoskopfunktionen.

Der so aufgebaute Endoskopschaft 1 (bestehend aus dem Anschlusseinsatz 4, dem biegeflexible Schaftabschnitt 2, ggf. dem aktiv abwinkelbaren Abschnitt und dem daran sich anschließenden Endoskopkopf - in dieser Reihenfolge) beinhaltet alle Funktionen des Endoskops sowie deren Anschlüsse 8, 10, 12 für das Anschließen an eine (nicht dargestellte) separate Betriebseinheit/-station. Eine solche Station umfasst u.a. Einrichtungen zum Betreiben der Optik und der Beleuchtung (ggf. einschließlich deren Kühlung), Einrichtungen für das Betätigen des Abwinklungsabschnitts, Einrichtungen (Pumpen) für das Einpressen von Spüllösungen oder Medikamenten und/oder das Absaugen von Flüssigkeiten, etc..

Aus diesem Grund ist es bevorzugt, den so definierten Endoskopschaft 1 als Ein-Weg-Artikel zu konzipieren, um aufwändige Reinigungsarbeiten zu vermeiden. Hier hat sich gezeigt, dass sowohl die Funktionen wie auch deren Anschlüsse preisgünstig hergestellt werden können, sodass eine Ein-Weg-Bauweise nicht nur aus hygienischen Gründen vorteilhaft ist sondern sich auch als wirtschaftlich erweist.

Das erfindungsgemäße Endoskop hat einen Handgriff 14, mittels dem das Endoskop/der Endoskopschaft 1 vorzugsweise gemäß vorstehender Definition in einer Endoskopie-Anwendung gehalten werden kann. Vorzugsweise sind im/am Handgriff 14 Betätigungselemente angeordnet, mittels denen die Funktionen des Endoskops aktuiert werden können.

Beispielsweise hat der Handgriff 14 ein Bedienerfeld 16 vorzugsweise in Form einer Tastatur, Joystick, Platte oder eines Touchscreen, die sich in ergonomisch günstiger Weise an einem Griffabschnitt 18 anschließt, derart, dass das Bedienerfeld 16 mit den Fingern der zur Greifhand zweiten Hand betätigt werden kann. Optional kann alternativ oder zusätzlich zu dem Bedienerfeld 16 ein (nicht weiter gezeigter) Lage-/G-Sensor/ Beschleunigungssensor bekannter Konstruktion integriert werden, um über z. B. eine Bewegung des Handgriffs die Endoskopspitze zu steuern.

Vorzugsweise ist der Griffabschnitt 18 als Halb- oder Drei-Viertelschale ausgebildet und definiert eine Art Klammer oder Manschette, die auf den Endoskopschaft 1 und vorzugsweise auf den Rohrlauf 4a des Anschlusseinsatzes 4 aufgesteckt werden kann. Hierfür hat der Griffabschnitt 18 beispielsweise eine Rohrform mit durchgehenden Längsschlitz 20, wobei im Längsschlitz 20 eine Anzahl von Zacken oder dergleichen Hinterschneidungen (Ausnehmungen) 22 ausgebildet sind. Die Rohrform ist gemäß diesem Ausführungsbeispiel im Querschnitt oval ausgebildet, um gut gehalten werden zu können und dem Greifer auch ein taktiles Gefühl für die Rotationslage des Endoskopschafts zu vermitteln. Es ist aber auch denkbar, den Griffabschnitt rund oder eckig auszubilden.

Der Anschlusseinsatz 4 hat im Bereich seines Rohrlaufs 4a ebenfalls eine Anzahl von Hinterschneidungen (Vorsprüngen) 24, die bei Einsetzen in den schalenförmigen Griffabschnitt 18 des Handgriffs 14 mit den dort ausgebildeten Hinterschneidungen 22 in Eingriff kommen und so die Relativposition des Handgriffs 14 zum Anschlusseinsatz 4 (und damit zum gesamten Endoskopschaft) festlegen.

Der Handgriff 14 bildet zumindest im Bereich des Bedienfeldes 16 ein Gehäuse 26, in dem elektrische/elektronische Bauteile zur Betätigung der separaten Betriebseinheit/- station untergebracht sind. Vorzugsweise betreffen diese elektrischen/elektronischen Bauteile eine kabellose Kommunikationseinrichtung sowie deren Energieversorgung. Als kabellose Kommunikationseinrichtung kommen beispielsweise W-Lan, Blue-Tooth oder Infrarot in Frage. Es ist aber auch denkbar, am Handgriff 14 eine Verkabelung anzuordnen, über die der Handgriff 14 (elektrisch) mit der Betriebseinheit/-station verbunden werden kann/ist.

Das Gehäuse 26 ist dabei fluiddicht oder aber mit einer fluiddichten Hülle (insbesondere im Bereich des Bedienfeldes 16) ummantelt, sodass dessen Oberfläche beispielsweise durch einfaches Eintauchen in eine Reinigungsflüssigkeit gesäubert werden kann.

Optional ist am Handgriff 14 im Bereich des Griffabschnitts 18 ein Sensor oder Schalter (nicht gezeigt) vorgesehen, der den Anschlusseinsatz 4 dann registriert, wenn dieser in den Handgriff 14 (dessen Griffabschnitt 18 mit Schalenform) eingesetzt ist. Der Schalter/Sensor ist mit der Elektronik im Handgriff 14 verbunden und schaltet diese nur dann frei, wenn der Handgriff 14 korrekt mit dem Endoskopschaft 1 mechanisch gekoppelt ist. So wird das Betätigen der Endoskopfunktionen bei nicht/falsch gekoppeltem Handgriff 14 verhindert. Weiter optional ist der Handgriff für Rechts- oder Linkshänder und für unterschiedliche Handgrößen konfigurierbar.

Gemäß der anliegenden Figuren ist das Bedienfeld 16 in Form einer Scheibe/Platte ausgebildet, die sich unter einem spitzen Winkel sowie seitlich zum Griffabschnitt 18 erstreckt. Auf diese Weise kann der Griffabschnitt 18 mit einer Hand (z.B. der rechten Hand) ergriffen und parallel dazu das Bedienfeld 16 mit der anderen Hand betätigt werden.

Schließlich ist aus den Figuren zu ersehen, dass bei in den Handgriff 14 (dessen Griffabschnitt 18) eingesetztem Anschlusseinsatz 4 die daran ausgebildeten Anschlüsse (Funktionsanschlüsse und Arbeitskanalanschlüsse/-zugänge) 8-12 aus dem Handgriff 14 (dessen Griffabschnitt 18) vorzugsweise proximal herausragen. Sie sind daher auch bei eingesetztem Zustand des Anschlusseinsatzes 4 von außen frei zugänglich. Es ist somit möglich, Anschlüsse nach Einsetzen des Anschlusseinsatzes 4 in den Handgriff 14 beliebig zu nutzen oder wieder zu verschließen.

## Patentansprüche

1. Endoskop mit einem Handgriff (14), einem Endoskopschaft (1), an dessen distalem Ende zumindest eine Optik sowie eine Beleuchtung vorgesehen sind, die über Funktions- oder Versorgungskanäle innerhalb des Endoskopschafts (1) an eine Betriebseinheit oder Betriebsstation angeschlossen oder anschließbar sind und einem am distalen Ende des Endoskopschafts (1) sich öffnenden Arbeitskanal (6), der im Endoskopschaft (1) ausgeformt und dafür angepasst ist, chirurgischen Instrumente der Minimalinvasivbauart längsverschieblich aufzunehmen, wobei der Arbeitskanal (6) am proximalen Endbereich des Endoskops zumindest zwei separate, unabhängig voneinander nutzbare Anschlüsse (10, 12) hat, von denen zumindest ein erster Anschluss (10) dazu geeignet und dazu angepasst ist, um darüber das chirurgische Instrument in den Arbeitskanal einzuführen und von denen zumindest ein zweiter Anschluss (12) dazu geeignet und dazu angepasst ist, um ein Fluid in den Arbeitskanal (6) proximal einzuleiten oder aus diesem zu entnehmen; wobei der Endoskopschaft (1) einen proximalen Anschlusseinsatz (4) hat, an dem die proximalen Arbeitskanal-Anschlüsse (10, 12) ausgebildet sind, wobei der Anschlusseinsatz (4) einen Rohrlauf (4a) hat, dessen eines Ende mit dem Arbeitskanal (6) innerhalb des mit dem Anschlusseinsatz (4) fest verbundenen biegeflexiblen Schaftabschnitts (2) fluidverbunden ist und an dessen anderem Ende ein Anschlussstutzen (4b) angeordnet ist mit einer Anzahl von Anschlussrohren, welche sämtlich in den Rohrlauf (4a) einmünden und welche die proximalen Arbeitskanal-Anschlüsse (10, 12) bilden; **dadurch gekennzeichnet, dass** die Funktions- oder Versorgungskanäle unter Umgehung eines Handgriffs (14) des Endoskops an die Betriebseinheit oder Betriebsstation angeschlossen oder anschließbar sind.

2. Endoskop nach Anspruch 1, **dadurch gekennzeichnet, dass** der zumindest eine erste Anschluss (10) einen Kanal bildet, dessen Durchmesser dem des Arbeitskanals (6) entspricht oder größer ist und dass der zumindest eine zweite Anschluss (12) ein Kopplungsmittel aufweist, das für das Ankoppeln einer Fluidquelle oder einer Absaugvorrichtung geeignet und angepasst ist.

3. Endoskop nach Anspruch 1 oder 2, **dadurch gekennzeichnet, dass** der zumindest eine erste Anschluss (10) axial oder im Wesentlichen axial zum Arbeitskanal (6) ausgerichtet ist und der zumindest eine zweite Anschluss (12) sich in einem Winkel zum Arbeitskanal (6) zwischen 30° und 120°, erstreckt.

4. Endoskop nach einem der vorstehenden Ansprüche, **dadurch gekennzeichnet, dass** der Endoskopschaft (1) zumindest einen biegeflexiblen Abschnitt (2) hat, der dazu geeignet und angepasst ist, bei einer Vorwärtsbewegung des Endoskops in einen Hohlraum eigenständig Hohlraumwindungen zu folgen und der Anschlusseinsatz (4) im Vergleich zum biegeflexiblen Abschnitt (2) starr ist.

5. Endoskop nach Anspruch 1 oder 4, **dadurch gekennzeichnet, dass** der Rohrlauf (4a) dafür angepasst ist, in einen Handgriff (14) des Endoskops eingesetzt zu werden, derart, dass die Anschlüsse (10, 12) des Anschlussstutzens (4b) zumindest abschnittsweise aus dem Handgriff (14) herausragen oder von außen zugänglich sind.

6. Endoskop nach Anspruch 5, **dadurch gekennzeichnet, dass** der Handgriff (14) als lösbarer Aufsteckhandgriff ausgebildet ist, der mit dem Anschlusseinsatz (4) zusammenwirkt.

7. Endoskop nach Anspruch 6, **dadurch gekennzeichnet, dass** der lösbare Aufsteckhandgriff nur mit dem Rohrlauf (4a) des Anschlusseinsatzes (4) zusammenwirkt.

8. Endoskop nach Anspruch 6, **dadurch gekennzeichnet, dass** der Handgriff (14) mit dem Anschlusseinsatz (4) klemmend zusammenwirkt.

9. Endoskop nach einem der Ansprüche 4 bis 8, **dadurch gekennzeichnet, dass** am Anschlusseinsatz (4) weitere Anschlüsse (8) für das Anschließen der Versorgungs-/Funktionskanäle an die Betriebseinheit oder Betriebsstation ausgebildet sind.

10. Endoskop nach einem der Ansprüche 5 bis 9, **dadurch gekennzeichnet, dass** der Handgriff (14) eine Betätigungseinrichtung (16) zur Aktuierung der Betriebseinheit oder Betriebsstation hat.

11. Endoskop nach Anspruch 10, **dadurch gekennzeichnet, dass** die Betätigungseinrichtung (16) ein Tastenfeld oder ein Touchscreen ist.

12. Endoskop nach Anspruch 10 oder 11, **dadurch gekennzeichnet, dass** die Betätigungseinrichtung (16) die Betriebseinheit oder die Betriebsstation kabellos aktuiert.

## Claims

1. Endoscope with a handle (14),
an endoscope shaft (1), at the distal end of which optics and lighting are provided that are connected or can be connected to an operating unit or an operating station via function or supply channels within the endoscope shaft (1), and a working channel (6) opening at the distal end of the endoscope shaft (1), which is formed within the endoscope shaft (1) and adapted to receive, in a manner so as to be longitudinally displaceable, surgical instruments of the minimally invasive type, the working channel (6) at the proximal end of the endoscope having at least two separate connections (10, 12) that can be used independently of one another, of which at least one first connection (10) is suited and adapted to introduce through it the surgical instrument into the working channel, and of which at least one second connection (12) is suited and adapted to proximally introduce a fluid into the working channel (6) or remove same therefrom, wherein
the endoscope shaft (1) has a proximal connection insert (4) on which the proximal working channel connections (10, 12) are formed, the connection insert (4) having a tubing (4a), one end of which is connected in a fluidal manner to the working channel (6) within the flexible shaft portion (2) firmly attached to the connection insert (4) and on the other end of which a connecting piece (4b) is arranged having a number of connecting tubes that all join the tubing (4a) and form the proximal working channel connections (10, 12), **characterized in that**
the function or supply channels are connected or can be connected to an operating unit or operating station, thereby bypassing a handle (14) of the endoscope.

2. The endoscope according to claim 1, **characterized in that** the at least one first connection (10) forms a channel, the diameter of which corresponds to that of the working channel (6) or is larger than the working channel (6), and that the at least one second connection (12) has coupling means suited and adapted for coupling a fluid source or a suction device.

3. The endoscope according to claim 1 or 2, **characterized in that** the at least one first connection (10) is oriented axially or substantially axially to the working channel (6) and that the at least one second connection (12) extends at an angle of 30° to 120° to the working channel (6).

4. The endoscope according to anyone of the preceding claims, **characterized in that** the endoscope shaft (1) has at least one flexible portion (2) suited and adapted, in case the endoscope is moved into a cavity in a forward direction, to independently follow windings in the cavity, and the connection insert (4) is rigid in comparison to the flexible portion (2).

5. The endoscope according to claim 1 or 4, **characterized in that** the tubing (4a) is adapted to be inserted in a handle (14) of the endoscope such that the connections (10, 12) of the connecting piece (4b) project from the handle (14) at least sectionally or are accessible from the outside.

6. The endoscope according to claim 5, **characterized in that** the handle (14) is formed as releasable slip-on handle acting together with the connection insert (4).

7. The endoscope according to claim 6, **characterized in that** the releasable slip-on handle only acts together with the tubing (4a) of the connection insert (4).

8. The endoscope according to claim 6, **characterized in that** the handle acts together with the connection insert (4) in a clamping manner.

9. The endoscope according to anyone of claims 4 to 8, **characterized in that** further connections (8) are formed on the connection insert (4) for connecting supply/functional channels to the operating unit or operating station.

10. The endoscope according to anyone of claims 5 to 9, **characterized in that** the handle (14) has an actuating device (16) for actuating the operating unit or operating station.

11. The endoscope according to claim 10, **characterized in that** the actuating device (16) is a keypad or a touchscreen.

12. The endoscope according to claim 10 or 11, **characterized in that** the actuating device (16) actuates the operating unit or the operating station in a wireless manner.

## Revendications

1. Endoscope avec une poignée (14),
un manche d'endoscope (1), au niveau de l'extrémité distale duquel au moins une optique et un éclairage sont prévus, qui sont raccordés ou peuvent être raccordés à une unité de service ou une station de service par l'intermédiaire de canaux fonctionnels ou de canaux d'alimentation à l'intérieur du manche d'endoscope (1), et un canal de travail (6) s'ouvrant au niveau de l'extrémité distale du manche d'endoscope (1), qui est formé dans le manche d'endoscope (1) et est adapté pour recevoir de manière à pouvoir coulisser longitudinalement des instruments chirurgicaux peu invasifs, dans lequel le canal de travail (6) a, au niveau de la zone d'extrémité proximale de l'endoscope, au moins deux raccords (10, 12) séparés, pouvant être utilisés indépendamment les uns des autres, parmi lesquels au moins un premier raccord (10) est approprié et adapté pour introduire par son intermédiaire l'instrument de chirurgie dans le canal de travail et parmi lesquels au moins un deuxième raccord (12) est approprié et adapté pour acheminer de manière proximale un fluide à l'intérieur du canal de travail (6) ou le prélever de ce dernier; dans lequel le manche d'endoscope (1) a un insert de raccordement (4) proximal, au niveau duquel les raccords de canal de travail (10, 12) proximaux sont réalisés, dans lequel l'insert de raccordement (4) a un tube en forme de canon (4a), dont une extrémité est reliée de manière fluidique au canal de travail (6) à l'intérieur de la section de manche (2) flexible reliée de manière solidaire à l'insert de raccordement (4) et au niveau de l'autre extrémité duquel une tubulure de raccordement (4b) est disposée avec un nombre donné de tubes de raccordement, qui débouchent tous dans le tube en forme de canon (4a) et lesquels forment les raccords de canal de travail (10, 12) proximaux ; **caractérisé en ce que**
les canaux fonctionnels ou d'alimentation sont raccordés ou peuvent être raccordés à l'unité de service ou la station de service en contournant une poignée (4) de l'endoscope.

2. Endoscope selon la revendication 1, **caractérisé en ce que** l'au moins un premier raccord (10) forme un canal, dont le diamètre correspond à celui du canal de travail (6) ou est plus grand, et que l'au moins un deuxième raccord (12) présente un moyen de couplage, qui est approprié et adapté pour l'accouplement d'une source de fluide ou d'un dispositif d'évacuation par aspiration.

3. Endoscope selon la revendication 1 ou 2, **caractérisé en ce que** l'au moins un premier raccord (10) est orienté de manière axiale ou de manière sensiblement axiale par rapport au canal de travail (6), et l'au moins un deuxième raccord (12) s'étend selon un angle par rapport au canal de travail (6) compris entre 30° et 120°.

4. Endoscope selon l'une quelconque des revendications précédentes, **caractérisé en ce que** le manche d'endoscope (1) a au moins une section (2) flexible, qui est appropriée et adaptée pour suivre en toute autonomie des méandres de cavité lors d'un déplacement vers l'avant de l'endoscope dans une cavité, et l'insert de raccordement (4) est rigide en comparaison avec la section (2) flexible.

5. Endoscope selon la revendication 1 ou 4, **caractérisé en ce que** le tube en forme de canon (4a) est adapté pour être inséré dans une poignée (14) de l'endoscope de telle manière que les raccords (10, 12) de la tubulure de raccordement (4b) dépassent au moins par endroits de la poignée (14) ou sont accessibles depuis l'extérieur.

6. Endoscope selon la revendication 5, **caractérisé en ce que** la poignée (14) est réalisée sous la forme d'une poignée à emboîter amovible, qui coopère avec l'insert de raccordement (4).

7. Endoscope selon la revendication 6, **caractérisé en ce que** la poignée à emboîter amovible coopère seulement avec le tube en forme de canon (4a) de l'insert de raccordement (4).

8. Endoscope selon la revendication 6, **caractérisé en ce que** la poignée (14) coopère par serrage avec l'insert de raccordement (4).

9. Endoscope selon l'une quelconque des revendications 4 à 8, **caractérisé en ce que** d'autres raccords (8) pour le raccordement des canaux d'alimentation/fonctionnels à l'unité de service ou la station de service sont réalisés au niveau de l'insert de raccordement (4).

10. Endoscope selon l'une quelconque des revendications 5 à 9, **caractérisé en ce que** la poignée (14) a un dispositif d'actionnement (16) servant à activer l'unité de service ou la station de service.

11. Endoscope selon la revendication 10, **caractérisé en ce que** le dispositif d'actionnement (16) est un pavé tactile ou un écran tactile.

12. Endoscope selon la revendication 10 ou 11, **caractérisé en ce que** le dispositif d'actionnement (16) active l'unité de service ou la station de service sans câble.
